# EUROPEAN PATENT SPECIFICATION

(11) **EP 0 657 468 B1**
(45) Date of publication and mention of the grant of the patent: **20.07.2005**
(21) Application number: 94116169.7
(22) Date of filing: 13.10.1994
(51) Int. Cl.: C07K 14/475, C12N 15/00, A61K 38/19

(54) **Human stromae derived factor**
Humaner aus Stroma erhältlicher Faktor
Facteur dérivé du stroma humain

(30) Priority: 14.10.1993 JP 28050593
(43) Date of publication of application: 14.06.1995
(60) Divisional application: 04005847.1
(73) Proprietor: ONO PHARMACEUTICAL CO., LTD., Osaka (JP)
(72) Inventor: Honjo,Tasuku, Sakyo-ku, Kyoto-shi, Kyoto (JP); Shirozu, Michio, Kyoto-shi, Kyoto (JP); Tada, Hideaki, c/o Minase Research Institute, Shimamotocho, Mishima-gun, Osaka (JP)
(74) Representative: Henkel, Feiler & Hänzel

(56) References cited:
- EP-A- 0 607 054
- PROC. NATL. ACAD. SCI. USA, vol.91, March 1994 pages 2305 - 2309 NAGASAWA, T. ET AL. 'Molecular cloning and structure of a pre-B-cell growth-stimulating factor'
- SCIENCE, vol.261, 30 July 1993 pages 600 - 603 TASHIRO, K. ET AL. 'Signal sequence trap: a cloning strategy for secreted proteins and type I membrane proteins'

### SEQUENCE LISTING

(1) GENERAL INFORMATION:
   (i) APPLICANT:
      (A) NAME: Ono Pharmaceutical Co., Ltd.
      (B) STREET: 1-5, Doshomachi 2-chome
      (C) CITY: Chuo-ku, Osaka-shi
      (D) STATE: Osaka
      (E) COUNTRY: Japan
      (F) POSTAL CODE (ZIP): 541
   (ii) TITLE OF INVENTION: Novel Polypeptides and DNAs encoding them
   (iii) NUMBER OF SEQUENCES: 20
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.25 (EPO)
(2) INFORMATION FOR SEQ ID NO:1:
   (i) SEQUENCE CHARACTERISTICS:
      (A) LENGTH: 89 amino acids
      (B) TYPE: amino acid
      (D) TOPOLOGY: linear
   (ii) MOLECULE TYPE: protein
   (xi) SEQUENCE DESCRIPTION: SEQ ID NO:1:
   Met Asn Ala Lys Val Val Val Val Leu Val Leu Val Leu Thr Ala Leu
   1               5                   10                  15
   Cys Leu Ser Asp Gly Lys Pro Val Ser Leu Ser Tyr Arg Cys Pro Cys
               20                  25                  30
   Arg Phe Phe Glu Ser His Val Ala Arg Ala Asn Val Lys His Leu Lys
           35                  40                  45
   Ile Leu Asn Thr Pro Asn Cys Ala Leu Gln Ile Val Ala Arg Leu Lys
       50                  55                  60
   Asn Asn Asn Arg Gln Val Cys Ile Asp Pro Lys Leu Lys Trp Ile Gln
   65                  70                  75                  80
   Glu Tyr Leu Glu Lys Ala Leu Asn Lys
                   85
(2) INFORMATION FOR SEQ ID NO:2:
   (i) SEQUENCE CHARACTERISTICS:
      (A) LENGTH: 267 base pairs
      (B) TYPE: nucleic acid
      (C) STRANDEDNESS: single
      (D) TOPOLOGY: linear
   (ii) MOLECULE TYPE: cDNA to mRNA
   (xi) SEQUENCE DESCRIPTION: SEQ ID NO:2:
   ATGAACGCCA AGGTCGTGGT CGTGCTGGTC CTCGTGCTGA CCGCGCTCTG CCTCAGCGAC    60
   GGGAAGCCCG TCAGCCTGAG CTACAGATGC CCATGCCGAT TCTTCGAAAG CCATGTTGCC   120
   AGAGCCAACG TCAAGCATCT CAAAATTCTC AACACTCCAA ACTGTGCCCT TCAGATTGTA   180
   GCCCGGCTGA AGAACAACAA CAGACAAGTG TGCATTGACC CGAAGCTAAA GTGGATTCAG   240
   GAGTACCTGG AGAAAGCTTT AAACAAG                                       267
(2) INFORMATION FOR SEQ ID NO:3:
   (i) SEQUENCE CHARACTERISTICS:
      (A) LENGTH: 1856 base pairs
      (B) TYPE: nucleic acid
      (C) STRANDEDNESS: single
      (D) TOPOLOGY: linear
   (ii) MOLECULE TYPE: cDNA to mRNA
   (xi) SEQUENCE DESCRIPTION: SEQ ID NO:3:
   TCTCCGTCAG CCGCATTGCC CGCTCGGCGT CCGGCCCCCG ACCCGTGCTC GTCCGCCCGC    60
   CCGCCCGCCC GCCCGCGCCA TGAACGCCAA GGTCGTGGTC GTGCTGGTCC TCGTGCTGAC   120
   CGCGCTCTGC CTCAGCGACG GGAAGCCCGT CAGCCTGAGC TACAGATGCC CATGCCGATT   180
   CTTCGAAAGC CATGTTGCCA GAGCCAACGT CAAGCATCTC AAAATTCTCA ACACTCCAAA   240
   CTGTGCCCTT CAGATTGTAG CCCGGCTGAA GAACAACAAC AGACAAGTGT GCATTGACCC   300
   GAAGCTAAAG TGGATTCAGG AGTACCTGGA GAAAGCTTTA AACAAGTAAG CACAACAGCC   360
   AAAAAGGACT TTCCGCTAGA CCCACTCGAG GAAAACTAAA ACCTTGTGAG AGATGAAAGG   420
   GCAAAGACGT GGGGGAGGGG GCCTTAACCA TGAGGACCAG GTGTGTGTGT GGGGTGGGCA   480
   CATTGATCTG GGATCGGGCC TGAGGTTTGC AGCATTTAGA CCCTGCATTT ATAGCATACG   540
   GTATGATATT GCAGCTTATA TTCATCCATG CCCTGTACCT GTGCACGTTG GAACTTTTAT    600
   TACTGGGGTT TTTCTAAGAA AGAAATTGTA TTATCAACAG CATTTTCAAG CAGTTAGTTC    660
   CTTCATGATC ATCACAATCA TCATCATTCT CATTCTCATT TTTTAAATCA ACGAGTACTT    720
   CAAGATCTGA ATTTGGCTTG TTTGGAGCAT CTCCTCTGCT CCCCTGGGGA GTCTGGGCAC    780
   AGTCAGGTGG TGGCTTAACA GGGAGCTGGA AAAAGTGTCC TTTCTTCAGA CACTGAGGCT    840
   CCCGCAGCAG CGCCCCTCCC AAGAGGAAGG CCTCTGTGGC ACTCAGATAC CGACTGGGGC    900
   TGGGGCGCCG CCACTGCCTT CACCTCCTCT TTCAAACCTC AGTGATTGGC TCTGTGGGCT    960
   CCATGTAGAA GCCACTATTA CTGGGACTGT CTCAGAGACC CCTCTCCCAG CTATTCCTAC   1020
   TCTCTCCCCG ACTCCGAGAG CATGCTTAAT CTTGCTTCTG CTTCTCATTT CTGTAGCCTG   1080
   ATCAGCGCCG CACCAGCCGG GAAGAGGGTG ATTGCTGGGG CTCGTGCCCT GCATCCCTCT   1140
   CCTCCCAGGG CCTGCCCCAC AGCTCGGGCC CTCTGTGAGA TCCGTCTTTG GCCTCCTCCA   1200
   GAATGGAGCT GGCCCTCTCC TGGGGATGTG TAATGGTCCC CCTGCTTACC CGCAAAAGAC   1260
   AAGTCTTTAC AGAATCAAAT GCAATTTTAA ATCTGAGAGC TCGCTTGAGT GACTGGGTTT   1320
   GTGATTGCCT CTGAAGCCTA TGTATGCCAT GGAGGCACTA ACAAACTCTG AGGTTTCCGA   1380
   AATCAGAAGC GAAAAAATCA GTGAATAAAC CATCATCTTG CCACTACCCC CTCCTGAAGC   1440
   CACAGCAGGG GTTCAGGTTC CAATCAGAAC TGTTGGCAAG GTGACATTTC CATGCATAGA   1500
   TGCGATCCAC AGAAGGTCCT GGTGGTATTT GTAACTTTTT GCAAGGCATT TTTTTATATA   1560
   TATTTTTGTG CACATTTTTT TTTACGATTC TTTAGAAAAC AAATGTATTT CAAAATATAT   1620
   TTATAGTCGA ACAAGTCATA TATATGAATG AGAGCCATAT GAATGTCAGT AGTTTATACT   1680
   TCTCTATTAT CTCAAACTAC TGGCAATTTG TAAAGAAATA TATATGATAT ATAAATGTGA   1740
   TTGCAGCTTT TCAATGTTAG CCACAGTGTA TTTTTTCACT TGTACTAAAA TTGTATCAAA   1800
   TGTGACATTA TATGCACTAG CAATAAAATG CTAATTGTTT CATGGTAAAA AAAAAA       1856
(2) INFORMATION FOR SEQ ID NO:4:
   (i) SEQUENCE CHARACTERISTICS:
      (A) LENGTH: 1856 base pairs
      (B) TYPE: nucleic acid
      (C) STRANDEDNESS: single
      (D) TOPOLOGY: linear
   (ii) MOLECULE TYPE: cDNA to mRNA
   (vi) ORIGINAL SOURCE:
      (A) ORGANISM: Homo sapiens
      (H) CELL LINE: FLEB14
   (ix) FEATURE:
      (A) NAME/KEY: CDS
      (B) LOCATION: 80..349
      (C) IDENTIFICATION METHOD: by similarity to some other pattern
   (ix) FEATURE:
      (A) NAME/KEY: sig_peptide
      (B) LOCATION: 80..142
      (C) IDENTIFICATION METHOD: by similarity with known sequence or to an established consensus
   (ix) FEATURE:
      (A) NAME/KEY: mat_peptide
      (B) LOCATION: 143..346
      (C) IDENTIFICATION METHOD: by similarity with known sequence or to an established consensus
   (xi) SEQUENCE DESCRIPTION: SEQ ID NO:4:
   TCTCCGTCAG CCGCATTGCC CGCTCGGCGT CCGGCCCCCG ACCCGTGCTC GTCCGCCCGC    60
   CCGCCCGCCC GCCCGCGCC ATG AAC GCC AAG GTC GTG GTC GTG CTG GTC CTC    112
                        Met Asn Ala Lys Val Val Val Val Leu Val Leu
                        -21 -20                 -15
   GTG CTG ACC GCG CTC TGC CTC AGC GAC GGG AAG CCC GTC AGC CTG AGC    160
   Val Leu Thr Ala Leu Cys Leu Ser Asp Gly Lys Pro Val Ser Leu Ser
   -10                 -5                   1                   5
   TAC AGA TGC CCA TGC CGA TTC TTC GAA AGC CAT GTT GCC AGA GCC AAC    208
   Tyr Arg Cys Pro Cys Arg Phe Phe Glu Ser His Val Ala Arg Ala Asn
                10                  15                  20
   GTC AAG CAT CTC AAA ATT CTC AAC ACT CCA AAC TGT GCC CTT CAG ATT    256
   Val Lys His Leu Lys Ile Leu Asn Thr Pro Asn Cys Ala Leu Gln Ile
            25                  30                  35
   GTA GCC CGG CTG AAG AAC AAC AAC AGA CAA GTG TGC ATT GAC CCG AAG    304
   Val Ala Arg Leu Lys Asn Asn Asn Arg Gln Val Cys Ile Asp Pro Lys
        40                  45                  50
   CTA AAG TGG ATT CAG GAG TAC CTG GAG AAA GCT TTA AAC AAG TAAGCACAAC    356
   Leu Lys Trp Ile Gln Glu Tyr Leu Glu Lys Ala Leu Asn Lys
    55                  60                  65
   AGCCAAAAAG GACTTTCCGC TAGACCCACT CGAGGAAAAC TAAAACCTTG TGAGAGATGA    416
   AAGGGCAAAG ACGTGGGGGA GGGGGCCTTA ACCATGAGGA CCAGGTGTGT GTGTGGGGTG    476
   GGCACATTGA TCTGGGATCG GGCCTGAGGT TTGCAGCATT TAGACCCTGC ATTTATAGCA    536
   TACGGTATGA TATTGCAGCT TATATTCATC CATGCCCTGT ACCTGTGCAC GTTGGAACTT    596
   TTATTACTGG GGTTTTTCTA AGAAAGAAAT TGTATTATCA ACAGCATTTT CAAGCAGTTA    656
   GTTCCTTCAT GATCATCACA ATCATCATCA TTCTCATTCT CATTTTTTAA ATCAACGAGT    716
   ACTTCAAGAT CTGAATTTGG CTTGTTTGGA GCATCTCCTC TGCTCCCCTG GGGAGTCTGG    776
   GCACAGTCAG GTGGTGGCTT AACAGGGAGC TGGAAAAAGT GTCCTTTCTT CAGACACTGA    836
   GGCTCCCGCA GCAGCGCCCC TCCCAAGAGG AAGGCCTCTG TGGCACTCAG ATACCGACTG    896
   GGGCTGGGGC GCCGCCACTG CCTTCACCTC CTCTTTCAAA CCTCAGTGAT TGGCTCTGTG    956
   GGCTCCATGT AGAAGCCACT ATTACTGGGA CTGTCTCAGA GACCCCTCTC CCAGCTATTC   1016
   CTACTCTCTC CCCGACTCCG AGAGCATGCT TAATCTTGCT TCTGCTTCTC ATTTCTGTAG   1076
   CCTGATCAGC GCCGCACCAG CCGGGAAGAG GGTGATTGCT GGGGCTCGTG CCCTGCATCC   1136
   CTCTCCTCCC AGGGCCTGCC CCACAGCTCG GGCCCTCTGT GAGATCCGTC TTTGGCCTCC   1196
   TCCAGAATGG AGCTGGCCCT CTCCTGGGGA TGTGTAATGG TCCCCCTGCT TACCCGCAAA   1256
   AGACAAGTCT TTACAGAATC AAATGCAATT TTAAATCTGA GAGCTCGCTT GAGTGACTGG   1316
   GTTTGTGATT GCCTCTGAAG CCTATGTATG CCATGGAGGC ACTAACAAAC TCTGAGGTTT   1376
   CCGAAATCAG AAGCGAAAAA ATCAGTGAAT AAACCATCAT CTTGCCACTA CCCCCTCCTG   1436
   AAGCCACAGC AGGGGTTCAG GTTCCAATCA GAACTGTTGG CAAGGTGACA TTTCCATGCA   1496
   TAGATGCGAT CCACAGAAGG TCCTGGTGGT ATTTGTAACT TTTTGCAAGG CATTTTTTTA   1556
   TATATATTTT TGTGCACATT TTTTTTTACG ATTCTTTAGA AAACAAATGT ATTTCAAAAT   1616
   ATATTTATAG TCGAACAAGT CATATATATG AATGAGAGCC ATATGAATGT CAGTAGTTTA   1676
   TACTTCTCTA TTATCTCAAA CTACTGGCAA TTTGTAAAGA AATATATATG ATATATAAAT   1736
   GTGATTGCAG CTTTTCAATG TTAGCCACAG TGTATTTTTT CACTTGTACT AAAATTGTAT   1796
   CAAATGTGAC ATTATATGCA CTAGCAATAA AATGCTAATT GTTTCATGGT AAAAAAAAAA   1856
(2) INFORMATION FOR SEQ ID NO:5:
   (i) SEQUENCE CHARACTERISTICS:
      (A) LENGTH: 93 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Met Asn Ala Lys Val Val Val Val Leu Val Leu Val Leu Thr Ala Leu
   1 5 10 15
   Cys Leu Ser Asp Gly Lys Pro Val Ser Leu Ser Tyr Arg Cys Pro Cys
   20 25 30
   Arg Phe Phe Glu Ser His Val Ala Arg Ala Asn Val Lys His Leu Lys
   35 40 45
   Ile Leu Asn Thr Pro Asn Cys Ala Leu Gln Ile Val Ala Arg Leu Lys
   50 55 60
   Asn Asn Asn Arg Gln Val Cys Ile Asp Pro Lys Leu Lys Trp Ile Gln
   65 70 75 80
   Glu Tyr Leu Glu Lys Ala Leu Asn Lys Arg Phe Lys Met
   85 90
(2) INFORMATION FOR SEQ ID NO:6:
   (i) SEQUENCE CHARACTERISTICS:
      (A) LENGTH: 279 base pairs
      (B) TYPE: nucleic acid
      (C) STRANDEDNESS: single
      (D) TOPOLOGY: linear
   (ii) MOLECULE TYPE: cDNA to mRNA
   (xi) SEQUENCE DESCRIPTION: SEQ ID NO:6:
   ATGAACGCCA AGGTCGTGGT CGTGCTGGTC CTCGTGCTGA CCGCGCTCTG CCTCAGCGAC 60
   GGGAAGCCCG TCAGCCTGAG CTACAGATGC CCATGCCGAT TCTTCGAAAG CCATGTTGCC 120
   AGAGCCAACG TCAAGCATCT CAAAATTCTC AACACTCCAA ACTGTGCCCT TCAGATTGTA 180
   GCCCGGCTGA AGAACAACAA CAGACAAGTG TGCATTGACC CGAAGCTAAA GTGGATTCAG 240
   GAGTACCTGG AGAAAGCTTT AAACAAGAGG TTCAAGATG 279
(2) INFORMATION FOR SEQ ID NO:7:
   (i) SEQUENCE CHARACTERISTICS:
      (A) LENGTH: 3526 base pairs
      (B) TYPE: nucleic acid
      (C) STRANDEDNESS: single
      (D) TOPOLOGY: linear
   (ii) MOLECULE TYPE: cDNA to mRNA
   (xi) SEQUENCE DESCRIPTION: SEQ ID NO:7:
   TCTCCGTCAG CCGCATTGCC CGCTCGGCGT CCGGCCCCCG ACCCGTGCTC GTCCGCCCGC 60
   CCGCCCGCCC GCCCGCGCCA TGAACGCCAA GGTCGTGGTC GTGCTGGTCC TCGTGCTGAC 120
   CGCGCTCTGC CTCAGCGACG GGAAGCCCGT CAGCCTGAGC TACAGATGCC CATGCCGATT 180
   CTTCGAAAGC CATGTTGCCA GAGCCAACGT CAAGCATCTC AAAATTCTCA ACACTCCAAA 240
   CTGTGCCCTT CAGATTGTAG CCCGGCTGAA GAACAACAAC AGACAAGTGT GCATTGACCC 300
   GAAGCTAAAG TGGATTCAGG AGTACCTGGA GAAAGCTTTA AACAAGAGGT TCAAGATGTG 360
   AGAGGGTCAG ACGCCTGAGG AACCCTTACA GTAGGAGCCC AGCTCTGAAA CCAGTGTTAG 420
   GGAAGGGCCT GCCACAGCCT CCCCTGCCAG GGCAGGGCCC CAGGCATTGC CAAGGGCTTT 480
   GTTTTGCACA CTTTGCCATA TTTTCACCAT TTGATTATGT AGCAAAATAC ATGACATTTA 540
   TTTTTCATTT AGTTTGATTA TTCAGTGTCA CTGGCGACAC GTAGCAGCTT AGACTAAGGC 600
   CATTATTGTA CTTGCCTTAT TAGAGTGTCT TTCCACGGAG CCACTCCTCT GACTCAGGGC 660
   TCCTGGGTTT TGTATTCTCT GAGCTGTGCA GGTGGGGAGA CTGGGCTGAG GGAGCCTGGC 720
   CCCATGGTCA GCCCTAGGGT GGAGAGCCAC CAAGAGGGAC GCCTGGGGGT GCCAGGACCA 780
   GTCAACCTGG GCAAAGCCTA GTGAAGGCTT CTCTCTGTGG GATGGGATGG TGGAGGGCCA 840
   CATGGGAGGC TCACCCCCTT CTCCATCCAC ATGGGAGCCG GGTCTGCCTC TTCTGGGAGG 900
   GCAGCAGGGC TACCCTGAGC TGAGGCAGCA GTGTGAGGCC AGGGCAGAGT GAGACCCAGC 960
   CCTCATCCCG AGCACCTCCA CATCCTCCAC GTTCTGCTCA TCATTCTCTG TCTCATCCAT 1020
   CATCATGTGT GTCCACGACT GTCTCCATGG CCCCGCAAAA GGACTCTCAG GACCAAAGCT 1080
   TTCATGTAAA CTGTGCACCA AGCAGGAAAT GAAAATGTCT TGTGTTACCT GAAAACACTG 1140
   TGCACATCTG TGTCTTGTGT GGAATATTGT CCATTGTCCA ATCCTATGTT TTTGTTCAAA 1200
   GCCAGCGTCC TCCTCTGTGA CCAATGTCTT GATGCATGCA CTGTTCCCCC TGTGCAGCCG 1260
   CTGAGCGAGG AGATGCTCCT TGGGCCCTTT GAGTGCAGTC CTGATCAGAG CCGTGGTCCT 1320
   TTGGGGTGAA CTACCTTGGT TCCCCCACTG ATCACAAAAA CATGGTGGGT CCATGGGCAG 1380
   AGCCCAAGGG AATTCGGTGT GCACCAGGGT TGACCCCAGA GGATTGCTGC CCCATCAGTG 1440
   CTCCCTCACA TGTCAGTACC TTCAAACTAG GGCCAAGCCC AGCACTGCTT GAGGAAAACA 1500
   AGCATTCACA ACTTGTTTTT GGTTTTTAAA ACCCAGTCCA CAAAATAACC AATCCTGGAC 1560
   ATGAAGATTC TTTCCCAATT CACATCTAAC CTCATCTTCT TCACCATTTG GCAATGCCAT 1620
   CATCTCCTGC CTTCCTCCTG GGCCCTCTCT GCTCTGCGTG TCACCTGTGC TTCGGGCCCT 1680
   TCCCACAGGA CATTTCTCTA AGAGAACAAT GTGCTATGTG AAGAGTAAGT CAACCTGCCT 1740
   GACATTTGGA GTGTTCCCCT CCCACTGAGG GCAGTCGATA GAGCTGTATT AAGCCACTTA   1800
   AAATGTTCAC TTTTGACAAA GGCAAGCACT TGTGGGTTTT TGTTTTGTTT TTCATTCAGT   1860
   CTTACGAATA CTTTTGCCCT TTGATTAAAG ACTCCAGTTA AAAAAAATTT TAATGAAGAA   1920
   AGTGGAAAAC AAGGAAGTCA AAGCAAGGAA ACTATGTAAC ATGTAGGAAG TAGGAAGTAA   1980
   ATTATAGTGA TGTAATCTTG AATTGTAACT GTTCGTGAAT TTAATAATCT GTAGGGTAAT   2040
   TAGTAACATG TGTTAAGTAT TTTCATAAGT ATTTCAAATT GGAGCTTCAT GGCAGAAGGC   2100
   AAACCCATCA ACAAAAATTG TCCCTTAAAC AAAAATTAAA ATCCTCAATC CAGCTATGTT   2160
   ATATTGAAAA AATAGAGCCT GAGGGATCTT TACTAGTTAT AAAGATACAG AACTCTTTCA   2220
   AAACCTTTTG AAATTAACCT CTCACTATAC CAGTATAATT GAGTTTTCAG TGGGGCAGTC   2280
   ATTATCCAGG TAATCCAAGA TATTTTAAAA TCTGTCACGT AGAACTTGGA TGTACCTGCC   2340
   CCCAATCCAT GAACCAAGAC CATTGAATTC TTGGTTGAGG AAACAAACAT GACCCTAAAT   2400
   CTTGACTACA GTCAGGAAAG GAATCATTTC TATTTCTCCT CCATGGGAGA AAATAGATAA   2460
   GAGTAGAAAC TGCAGGGAAA ATTATTTGCA TAACAATTCC TCTACTAACA ATCAGCTCCT   2520
   TCCTGGAGAC TGCCCAGCTA AAGCAATATG CATTTAAATA CAGTCTTCCA TTTGCAAGGG   2580
   AAAAGTCTCT TGTAATCCGA ATCTCTTTTT GCTTTCGAAC TGCTAGTCAA GTGCGTCCAC   2640
   GAGCTGTTTA CTAGGGATCC CTCATCTGTC CCTCCGGGAC CTGGTGCTGC CTCTACCTGA   2700
   CACTCCCTTG GGCTCCCTGT AACCTCTTCA GAGGCCCTCG CTGCCAGCTC TGTATCAGGA   2760
   CCCAGAGGAA GGGGCCAGAG GCTCGTTGAC TGGCTGTGTG TTGGGATTGA GTCTGTGCCA   2820
   CGTGTATGTG CTGTGGTGTG TCCCCCTCTG TCCAGGCACT GAGATACCAG CGAGGAGGCT   2880
   GCAGAGGGCA CTCTGCTTGT TATTAGAGAT TACCTCCTGA GAAAAAAGCT TCCGCTTGGA   2940
   GCAGAGGGGC TGAATAGCAG AAGGTTGCAC CTCCCCCAAC CTTAGATGTT CTAAGTCTTT   3000
   CCATTGGATC TCATTGGACC CTTCCATGGT GTGATCGTCT GACTGGTGTT ATCACCGTGG   3060
   GCTCCCTGAC TGGGAGTTGA TCGCCTTTCC CAGGTGCTAC ACCCTTTTCC AGCTGGATGA   3120
   GAATTTGAGT GCTCTGATCC CTCTACAGAG CTTCCCTGAC TCATTCTGAA GGAGCCCCAT   3180
   TCCTGGGAAA TATTCCCTAG AAACTTCCAA ATCCCCTAAG CAGACCACTG ATAAAACCAT   3240
   GTAGAAAATT TGTTATTTTG CAACCTCGCT GGACTCTCAG TCTCTGAGCA GTGAATGATT   3300
   CAGTGTTAAA TGTGATGAAT ACTGTATTTT GTATTGTTTC AAGTGCATCT CCCAGATAAT   3360
   GTGAAAATGG TCCAGGAGAA GGCCAATTCC TATACGCAGC GTGCTTTAAA AAATAAATAA   3420
   GAAACAACTC TTTGAGAAAC AACAATTTCT ACTTTGAAGT CATACCAATG AAAAAATGTA   3480
   TATGCACTTA TAATTTTCCT AATAAAGTTC TGTACTCAAA TGTAAA                 3526
(2) INFORMATION FOR SEQ ID NO:8:
   (i) SEQUENCE CHARACTERISTICS:
      (A) LENGTH: 3526 base pairs
      (B) TYPE: nucleic acid
      (C) STRANDEDNESS: single
      (D) TOPOLOGY: linear
   (ii) MOLECULE TYPE: cDNA to mRNA
   (vi) ORIGINAL SOURCE:
      (A) ORGANISM: Homo sapiens
      (H) CELL LINE: FLEB14
   (ix) FEATURE:
      (A) NAME/KEY: CDS
      (B) LOCATION: 80..361
      (C) IDENTIFICATION METHOD: by similarity to some other pattern
   (ix) FEATURE:
      (A) NAME/KEY: sig_peptide
      (B) LOCATION: 80..142
      (C) IDENTIFICATION METHOD: by similarity with known sequence or to an established consensus
   (ix) FEATURE:
      (A) NAME/KEY: mat_peptide
      (B) LOCATION: 143..358
      (C) IDENTIFICATION METHOD: by similarity with known sequence or to an established consensus
   (xi) SEQUENCE DESCRIPTION: SEQ ID NO:8:
   TCTCCGTCAG CCGCATTGCC CGCTCGGCGT CCGGCCCCCG ACCCGTGCTC GTCCGCCCGC     60
   CCGCCCGCCC GCCCGCGCC ATG AAC GCC AAG GTC GTG GTC GTG CTG GTC CTC    112
                        Met Asn Ala Lys Val Val Val Val Leu Val Leu
                        -21 -20                 -15
   GTG CTG ACC GCG CTC TGC CTC AGC GAC GGG AAG CCC GTC AGC CTG AGC     160
   Val Leu Thr Ala Leu Cys Leu Ser Asp Gly Lys Pro Val Ser Leu Ser
   -10                 -5                  1                   5
   TAC AGA TGC CCA TGC CGA TTC TTC GAA AGC CAT GTT GCC AGA GCC AAC     208
   Tyr Arg Cys Pro Cys Arg Phe Phe Glu Ser His Val Ala Arg Ala Asn
               10                  15                  20
   GTC AAG CAT CTC AAA ATT CTC AAC ACT CCA AAC TGT GCC CTT CAG ATT     256
   Val Lys His Leu Lys Ile Leu Asn Thr Pro Asn Cys Ala Leu Gln Ile
           25                  30                  35
   GTA GCC CGG CTG AAG AAC AAC AAC AGA CAA GTG TGC ATT GAC CCG AAG      304
   Val Ala Arg Leu Lys Asn Asn Asn Arg Gln Val Cys Ile Asp Pro Lys
        40                  45                      50
   CTA AAG TGG ATT CAG GAG TAC CTG GAG AAA GCT TTA AAC AAG AGG TTC      352
   Leu Lys Trp Ile Gln Glu Tyr Leu Glu Lys Ala Leu Asn Lys Arg Phe
    55                      60                  65                  70
   AAG ATG TGAGAGGGTC AGACGCCTGA GGAACCCTTA CAGTAGGAGC CCAGCTCTGA      408
   Lys Met
   AACCAGTGTT AGGGAAGGGC CTGCCACAGC CTCCCCTGCC AGGGCAGGGC CCCAGGCATT    468
   GCCAAGGGCT TTGTTTTGCA CACTTTGCCA TATTTTCACC ATTTGATTAT GTAGCAAAAT    528
   ACATGACATT TATTTTTCAT TTAGTTTGAT TATTCAGTGT CACTGGCGAC ACGTAGCAGC    588
   TTAGACTAAG GCCATTATTG TACTTGCCTT ATTAGAGTGT CTTTCCACGG AGCCACTCCT    648
   CTGACTCAGG GCTCCTGGGT TTTGTATTCT CTGAGCTGTG CAGGTGGGGA GACTGGGCTG    708
   AGGGAGCCTG GCCCCATGGT CAGCCCTAGG GTGGAGAGCC ACCAAGAGGG ACGCCTGGGG    768
   GTGCCAGGAC CAGTCAACCT GGGCAAAGCC TAGTGAAGGC TTCTCTCTGT GGGATGGGAT    828
   GGTGGAGGGC CACATGGGAG GCTCACCCCC TTCTCCATCC ACATGGGAGC CGGGTCTGCC    888
   TCTTCTGGGA GGGCAGCAGG GCTACCCTGA GCTGAGGCAG CAGTGTGAGG CCAGGGCAGA    948
   GTGAGACCCA GCCCTCATCC CGAGCACCTC CACATCCTCC ACGTTCTGCT CATCATTCTC   1008
   TGTCTCATCC ATCATCATGT GTGTCCACGA CTGTCTCCAT GGCCCCGCAA AAGGACTCTC   1068
   AGGACCAAAG CTTTCATGTA AACTGTGCAC CAAGCAGGAA ATGAAAATGT CTTGTGTTAC   1128
   CTGAAAACAC TGTGCACATC TGTGTCTTGT GTGGAATATT GTCCATTGTC CAATCCTATG   1188
   TTTTTGTTCA AAGCCAGCGT CCTCCTCTGT GACCAATGTC TTGATGCATG CACTGTTCCC   1248
   CCTGTGCAGC CGCTGAGCGA GGAGATGCTC CTTGGGCCCT TTGAGTGCAG TCCTGATCAG   1308
   AGCCGTGGTC CTTTGGGGTG AACTACCTTG GTTCCCCCAC TGATCACAAA AACATGGTGG   1368
   GTCCATGGGC AGAGCCCAAG GGAATTCGGT GTGCACCAGG GTTGACCCCA GAGGATTGCT   1428
   GCCCCATCAG TGCTCCCTCA CATGTCAGTA CCTTCAAACT AGGGCCAAGC CCAGCACTGC   1488
   TTGAGGAAAA CAAGCATTCA CAACTTGTTT TTGGTTTTTA AAACCCAGTC CACAAAATAA   1548
   CCAATCCTGG ACATGAAGAT TCTTTCCCAA TTCACATCTA ACCTCATCTT CTTCACCATT   1608
   TGGCAATGCC ATCATCTCCT GCCTTCCTCC TGGGCCCTCT CTGCTCTGCG TGTCACCTGT   1668
   GCTTCGGGCC CTTCCCACAG GACATTTCTC TAAGAGAACA ATGTGCTATG TGAAGAGTAA   1728
   GTCAACCTGC CTGACATTTG GAGTGTTCCC CTCCCACTGA GGGCAGTCGA TAGAGCTGTA 1788
   TTAAGCCACT TAAAATGTTC ACTTTTGACA AAGGCAAGCA CTTGTGGGTT TTTGTTTTGT 1848
   TTTTCATTCA GTCTTACGAA TACTTTTGCC CTTTGATTAA AGACTCCAGT TAAAAAAAAT 1908
   TTTAATGAAG AAAGTGGAAA ACAAGGAAGT CAAAGCAAGG AAACTATGTA ACATGTAGGA 1968
   AGTAGGAAGT AAATTATAGT GATGTAATCT TGAATTGTAA CTGTTCGTGA ATTTAATAAT 2028
   CTGTAGGGTA ATTAGTAACA TGTGTTAAGT ATTTTCATAA GTATTTCAAA TTGGAGCTTC 2088
   ATGGCAGAAG GCAAACCCAT CAACAAAAAT TGTCCCTTAA ACAAAAATTA AAATCCTCAA 2148
   TCCAGCTATG TTATATTGAA AAAATAGAGC CTGAGGGATC TTTACTAGTT ATAAAGATAC 2208
   AGAACTCTTT CAAAACCTTT TGAAATTAAC CTCTCACTAT ACCAGTATAA TTGAGTTTTC 2268
   AGTGGGGCAG TCATTATCCA GGTAATCCAA GATATTTTAA AATCTGTCAC GTAGAACTTG 2328
   GATGTACCTG CCCCCAATCC ATGAACCAAG ACCATTGAAT TCTTGGTTGA GGAAACAAAC 2388
   ATGACCCTAA ATCTTGACTA CAGTCAGGAA AGGAATCATT TCTATTTCTC CTCCATGGGA 2448
   GAAAATAGAT AAGAGTAGAA ACTGCAGGGA AAATTATTTG CATAACAATT CCTCTACTAA 2508
   CAATCAGCTC CTTCCTGGAG ACTGCCCAGC TAAAGCAATA TGCATTTAAA TACAGTCTTC 2568
   CATTTGCAAG GGAAAAGTCT CTTGTAATCC GAATCTCTTT TTGCTTTCGA ACTGCTAGTC 2628
   AAGTGCGTCC ACGAGCTGTT TACTAGGGAT CCCTCATCTG TCCCTCCGGG ACCTGGTGCT 2688
   GCCTCTACCT GACACTCCCT TGGGCTCCCT GTAACCTCTT CAGAGGCCCT CGCTGCCAGC 2748
   TCTGTATCAG GACCCAGAGG AAGGGGCCAG AGGCTCGTTG ACTGGCTGTG TGTTGGGATT 2808
   GAGTCTGTGC CACGTGTATG TGCTGTGGTG TGTCCCCCTC TGTCCAGGCA CTGAGATACC 2868
   AGCGAGGAGG CTCCAGAGGG CACTCTGCTT GTTATTAGAG ATTACCTCCT GAGAAAAAAG 2928
   CTTCCGCTTG GAGCAGAGGG GCTGAATAGC AGAAGGTTGC ACCTCCCCCA ACCTTAGATG 2988
   TTCTAAGTCT TTCCATTGGA TCTCATTGGA CCCTTCCATG GTGTGATCGT CTGACTGGTG 3048
   TTATCACCGT GGGCTCCCTG ACTGGGAGTT GATCGCCTTT CCCAGGTGCT ACACCCTTTT 3108
   CCAGCTGGAT GAGAATTTGA GTGCTCTGAT CCCTCTACAG AGCTTCCCTG ACTCATTCTG 3168
   AAGGAGCCCC ATTCCTGGGA AATATTCCCT AGAAACTTCC AAATCCCCTA AGCAGACCAC 3228
   TGATAAAACC ATGTAGAAAA TTTGTTATTT TGCAACCTCG CTGGACTCTC AGTCTCTGAG 3288
   CAGTGAATGA TTCAGTGTTA AATGTGATGA ATACTGTATT TTGTATTGTT TCAAGTGCAT 3348
   CTCCCAGATA ATGTGAAAAT GGTCCAGGAG AAGGCCAATT CCTATACGCA GCGTGCTTTA 3408
   AAAAATAAAT AAGAAACAAC TCTTTGAGAA ACAACAATTT CTACTTTGAA GTCATACCAA 3468
   TGAAAAAATG TATATGCACT TATAATTTTC CTAATAAAGT TCTGTACTCA AATGTAAA 3526
(2) INFORMATION FOR SEQ ID NO:9:
   (i) SEQUENCE CHARACTERISTICS:
      (A) LENGTH: 1797 base pairs
      (B) TYPE: nucleic acid
      (C) STRANDEDNESS: single
      (D) TOPOLOGY: linear
   (ii) MOLECULE TYPE: cDNA to mRNA
   (xi) SEQUENCE DESCRIPTION: SEQ ID NO:9:
   GACCACTTTC CCTCTCGGTC CACCTCGGTG TCCTCTTGCT GTCCAGCTCT GCAGCCTCCG     60
   GCGCGCCCTC CCGCCCACGC CATGGACGCC AAGGTCGTCG CCGTGCTGGC CCTGGTGCTG    120
   GCCGCGCTCT GCATCAGTGA CGGTAAACCA GTCAGCCTGA GCTACCGATG CCCCTGCCGG    180
   TTCTTCGAGA GCCACATCGC CAGAGCCAAC GTCAAGCATC TGAAAATCCT CAACACTCCA    240
   AACTGTGCCC TTCAGATTGT TGCACGGCTG AAGAACAACA ACAGACAAGT GTGCATTGAC    300
   CCGAAATTAA AGTGGATCCA AGAGTACCTG GAGAAAGCTT TAAACAAGTA AGCACAACAG    360
   CCCAAAGGAC TTTCCAGTAG ACCCCCGAGG AAGGCTGACA TCCGTGGGAG ATGCAAGGGC    420
   AGTGGTGGGG AGGAGGGCCT GAACCCTGGC CAGGATGGCC GGCGGGACAG CACTGACTGG    480
   GGTCATGCTA AGGTTTGCCA GCATAAAGAC ACTCCGCCAT AGCATATGGT ACGATATTGC    540
   AGCTTATATT CATCCCTGCC CTCGCCCGTG CACAATGGAG CTTTTATAAC TGGGGTTTTT    600
   CTAAGGAATT GTATTACCCT AACCAGTTAG CTTCATCCCC ATTCTCCTCA TCCTCATCTT    660
   CATTTTAAAA AGCAGTGATT ACTTCAAGGG CTGTATTCAG TTTGCTTTGG AGCTTCTCTT    720
   TGCCCTGGGG CCTCTGGGCA CAGTTATAGA CGGTGGCTTT GCAGGGAGCC CTAGAGAGAA    780
   ACCTTCCACC AGAGCAGAGT CCGAGGAACG CTGCAGGGCT TGTCCTGCAG GGGGCGCTCC    840
   TCGACAGATG CCTTGTCCTG AGTCAACACA AGATCCGGCA GAGGGAGGCT CCTTTATCCA    900
   GTTCAGTGCC AGGGTCGGGA AGCTTCCTTT AGAAGTGATC CCTGAAGCTG TGCTCAGAGA    960
   CCCTTTCCTA GCCGTTCCTG CTCTCTGCTT GCCTCCAAAC GCATGCTTCA TCTGACTTCC   1020
   GCTTCTCACC TCTGTAGCCT GACGGACCAA TGCTGCAATG GAAGGGAGGA GAGTGATGTG   1080
   GGGTGCCCCC TCCCTCTCTT CCCTTTGCTT TCCTCTCACT TGGGCCCTTT GTGAGATTTT   1140
   TCTTTGGCCT CCTGTAGAAT GGAGCCAGAC CATCCTGGAT AATGTGAGAA CATGCCTAGA 1200
   TTTACCCACA AAACACAAGT CTGAGAATTA ATCATAAACG GAAGTTTAAA TGAGGATTTG 1260
   GACCTTGGTA ATTGTCCCTG AGTCCTATAT ATTTCAACAG TGGCTCTATG GGCTCTGATC 1320
   GAATATCAGT GATGAAAATA ATAATAATAA TAATAATAAC GAATAAGCCA GAATCTTGCC 1380
   ATGAAGCCAC AGTGGGGATT CTGGGTTCCA ATCAGAAATG GAGACAAGAT AAAACTTGCA 1440
   TACATTCTTA TGATCACAGA CGGCCCTGGT GGTTTTTGGT AACTATTTAC AAGGCATTTT 1500
   TTTACATATA TTTTTGTGCA CTTTTTATGT TTCTTTGGAA GACAAATGTA TTTCAGAATA 1560
   TATTTGTAGT CAATTCATAT ATTTGAAGTG GAGCCATAGT AATGCCAGTA GATATCTCTA 1620
   TGATCTTGAG CTACTGGCAA CTTGTAAAGA AATATATATG ACATATAAAT GTATTGTAGC 1680
   TTTCCGGTGT CAGCCACGGT GTATTTTTCC ACTTGGAATG AAATTGTATC AACTGTGACA 1740
   TTATATGCAC TAGCAATAAA ATGCTAATTG TTTCATGCTG TAAAAAAAAA AAAAAAA 1797
(2) INFORMATION FOR SEQ ID NO:10:
   (i) SEQUENCE CHARACTERISTICS:
      (A) LENGTH: 14 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:10:
   AATTCGCGGC CGCT 14
(2) INFORMATION FOR SEQ ID NO:11:
   (i) SEQUENCE CHARACTERISTICS:
      (A) LENGTH: 10 base pairs
      (B) TYPE: nucleic acid
      (C) STRANDEDNESS: double
      (D) TOPOLOGY: linear
   (ii) MOLECULE TYPE: cDNA
   (ix) FEATURE:
      (A) NAME/KEY: misc_feature
      (B) LOCATION: 1
      (D) OTHER INFORMATION: /label= phosphorylated
   (xi) SEQUENCE DESCRIPTION: SEQ ID NO:11:
   AGCGGCCGCG 10
(2) INFORMATION FOR SEQ ID NO:12:
   (i) SEQUENCE CHARACTERISTICS:
      (A) LENGTH: 28 base pairs
      (B) TYPE: nucleic acid
      (C) STRANDEDNESS: double
      (D) TOPOLOGY: linear
   (ii) MOLECULE TYPE: DNA (synthetic)
   (xi) SEQUENCE DESCRIPTION: SEQ ID NO:12:
   GTAATACGAC TCACTATAGG GGAGAGCT 28
(2) INFORMATION FOR SEQ ID NO:13:
   (i) SEQUENCE CHARACTERISTICS:
      (A) LENGTH: 28 base pairs
      (B) TYPE: nucleic acid
      (C) STRANDEDNESS: double
      (D) TOPOLOGY: linear
   (ii) MOLECULE TYPE: DNA (synthetic)
   (xi) SEQUENCE DESCRIPTION: SEQ ID NO:13:
   CTCCCCTATA GTGAGTCGTA TTACTGCA                                    28
(2) INFORMATION FOR SEQ ID NO:14:
   (i) SEQUENCE CHARACTERISTICS:
      (A) LENGTH: 32 base pairs
      (B) TYPE: nucleic acid
      (C) STRANDEDNESS: double
      (D) TOPOLOGY: linear
   (ii) MOLECULE TYPE: DNA (synthetic)
   (xi) SEQUENCE DESCRIPTION: SEQ ID NO:14:
   CTAGTCTATA GTGTCACCTA AATCGTGGGT AC   32
(2) INFORMATION FOR SEQ ID NO:15:
   (i) SEQUENCE CHARACTERISTICS:
      (A) LENGTH: 23 base pairs
      (B) TYPE: nucleic acid
      (C) STRANDEDNESS: double
      (D) TOPOLOGY: linear
   (ii) MOLECULE TYPE: DNA (synthetic)
   (xi) SEQUENCE DESCRIPTION: SEQ ID NO:15:
   CACGATTTAG GTGACACTAT AGA 23
(2) INFORMATION FOR SEQ ID NO:16:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:16:
   AATATAGTCG ACCACCATGA ACGCCAAGGT CGTGGTCGTG CTGG 44
(2) INFORMATION FOR SEQ ID NO:17:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:17:
   CGGCGGACTA GTTTACTTGT TTAAAGCTTT CTCCAGG 37
(2) INFORMATION FOR SEQ ID NO:18:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:18:
   GCCGCCACTA GTTTAGTGGT GGTGGTGGTG GTGCTTGTTT AAAGCTTTCT CCAGG 55
(2) INFORMATION FOR SEQ ID NO:19:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:19:
   CGGCGGACTA GTTCACATCT TGAACCTCTT GTTTAAAGC 39
(2) INFORMATION FOR SEQ ID NO:20:
   (i) SEQUENCE CHARACTERISTICS:
      (A) LENGTH: 57 base pairs
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:20:
   GCCGCCACTA GTTCAGTGGT GGTGGTGGTG GTGCATCTTG AACCTCTTGT TTAAAGC 57

## Description

### Field of the Invention

The present invention is related to novel polypeptides produced by human pro-B cell line and DNAs encoding them.

EP-A-0 607 054, which is a prior-art document under Article 54(3) and (4) EPC, discloses a process for constructing a cDNA library which has a selectivity for signal peptides, that makes it possible to efficiently identify unknown and useful polypeptide comprising a signal peptide. A novel polypeptide consisting of 89 amino acids (including a signal peptide) produced by a stroma cell line, which is useful as an agent for preventing or treating, for example, anemia, leukopenia or infections and the like, and DNAs coding for said polypeptide, have been identified using said process.

Science 261, pp. 600 - 603 (1993) discloses a method to clone, without the use of specific functional assays, complementary DNAs (cDNAs) that carry specific amino-terminal signal sequences, such as those encoding intercellular signal-transducing molecules and receptors. The vector used in this system directed the cell surface expression of interleukin-2 receptor fusion proteins when inserts with signal sequences were cloned in-frame with the correct orientation. An expression cDNA library was constructed from a bone marrow stromal cell line, which contained 5' portion-enriched cDNAs (the average size was 400 base pairs). Two cDNAs that encoded putative cytokine molecules, stromal cell-derived factor-1α (SDF-1α) and SDF-1β, which belong to the intercrine-macrophage inflammatory protein superfamily, were cloned.

In Proc. Natl. Acad. Sci., USA, Vol. 91, pp. 2305-2309 (1994) it is taught that generation and proliferation of early B-cell progenitors have been known to require stromal cell-derived molecules. A stromal cell line, PA6, was found to produce a soluble mediator, which was distinct from interleukin 7 (IL-7) and stem cell factor and supported the proliferation of a stromal cell-dependent pre-B-cell clone, DW34. A cDNA clone encoding this DW34 growth-stimulating factor was isolated by expression cloning. The nucleotide sequence contained a single substantial open reading frame of 267 nucleotides encoding an 89-amino acid polypeptide. The amino acid sequence of this cytokine, designated pre-B-cell growth-stimulating factor (PBSF), revealed that it is a member of intercrine α subfamily. Recombinant PBSF stimulated the proliferation of DW34 cells for itself and, furthermore, synergistically augmented the growth of DW34 as well as bone marrow B-cell progenitors in the presence of IL-7.

### Purpose of the Invention

The present invention is related to novel polypeptides produced by hematopoietic cells and DNAs encoding them. It is known that many kinds of growth and differentiation factors such as interleukin (IL) from hematopoietic cells.

This fact suggests that factors having similar or novel functions might be secreted therefrom in addition to the known secretly factors already found.

The present inventors have paid their attention to this point and attempted to find out a novel factors (polypeptide) produced from hematopoietic cells. The present inventors were screened by cross hybridization using mouse SDF-1 (Stromal Derived Factor 1; described in Japanese Patent Application No. 5-22098) cDNA as a probe and thus obtained human SDF-1 (2 kinds, α and β) produced from human pro-B cells and then completed the present invention.

When polypeptides having sequences identical or highly homologous with that of the polypeptide of the present invention and the DNAs encoding them are searched for with a computer, none is found out. Thus it has been proved that the polypeptide of the present invention and the DNA coding for the same are novel ones.
(1) a polypeptide having an amino acid sequence shown in SEQ ID NO. 1
(2) a DNA having a nucleotide sequence shown in SEQ ID NO. 2, and
(3) a DNA having a nucleotide sequence shown in SEQ ID NO. 3
(4) a polypeptide having an amino acid sequence shown in SEQ ID NO. 5
(5) a DNA having a nucleotide sequence shown in SEQ ID NO. 6, and
(6) a DNA having a nucleotide sequence shown in SEQ ID NO. 7

The present invention is concerned with polypeptides having the amino acid shown in SEQ ID. No. 1 or 5, and DNA encoding such a polypeptide. More particularly, the present invention is related to DNA having the nucleotide sequence shown in SEQ ID No. 2 or 3, and 6 or 7.

A further embodiment of the invention provides replication and expression vectors comprising DNA according to the invention. The vectors may be, for example, plasmid, virus or phage vectors provided with an origin of replication, optionally a promoter for the expression of the said DNA and optionally a regulator of the promoter. The vector may contain one or more selectable marker genes, for example an anpicillin resistance gene. The vector may be used in vitro, for example of the production of RNA corresponding to the DNA, or used to transfect or transform a host cell.

A further embodiment of the invention provides host cells transformed or transfected with the vectors for the replication and expression of DNA according to the invention, including the DNA SEQ. ID No. 2 or 3, and 6 or 7 or the open reading frame thereof. The cells will be chosen to be compatible with the vector and may for example be bacterial, yeast, insect or mammalian.

A further embodiment of the invention provides a method of producing a polypeptide which comprises culturing host cells of the present invention under conditions effective to express a polypeptide of the invention. Preferably, in addition, such a method is carried out under conditions in which the polypeptide of the invention is expressed and then produced from the host cells.

DNA according to the invention may also be inserted into the vectors described above in an antisense orientation in order to proved for the production of antisense RNA. Antisense RNA may also be produced by synthetic means. Such antisense RNA may be used in a method of controlling the levels of a polypeptide of the invention in a cell.

There are described monoclonal or polyclonal antibodies to a polypeptide according to the invention. Furthermore, there is described a process for the production of monoclonal or polyclonal antibodies to the polypeptides of the invention. Monoclonal antibodies may be prepared by conventional hybridoma technology using a polypeptide of the invention or a fragment thereof, as an immunogen. Polyclonal antibodies may also be prepared by conventional means which comprise inoculating a host animal, for example a rat or a rabbit, with a polypeptide of the invention and recovering immune serum.

The present invention also provides pharmaceutical compositions containing a polypeptide of the invention, in association with a pharmaceutically acceptable diluent and/or carrier.

The polypeptide of the present invention includes that which a part of their amino acid sequence is lacking (e.g., a polypeptide comprised of the only essential sequence for revealing a biological activity in an amino acid sequence shown in SEQ ID No. 1 or 5), that which a part of their amino acid sequence is replaced by other amino acids (e.g., those replaced by an amino acid having a similar property) and that which other amino acids are added or inserted into a part of their amino acid sequence, as well as those having the amino acid sequence shown in SEQ ID NO. 1 or 5.

As known well, there are one to six kinds of codon as that encoding one amino acid (for example, one kind of codon for Met, and six kinds of codon for leucine (Leu) are known). Accordingly, the nucleotide sequence of DNA can be changed in order to encode the polypeptide having the same amino acid sequence.

The DNA specified in (2) and (5) are sequence in the natural form.

The DNA shown in (3) and (6) indicate the sequence of the DNA specified in (2) and (5) with a untranslated region.

A signal peptide is a hydrophobic region located immediately downstream of the translation initiation amino acid Met. It is assumed that the signal peptide in the polypeptide of the present invention resides in a region ranging from Met at the 1-position to Gly at the 21-position in the amino acid sequence represented by Seq. ID No. 1 or 5. The region essentially responsible for the expression of the biological activity corresponds to the part of the amino acid sequences of the Seq. ID. No. 1 and 5 lacking of the signal peptides, i.e. the mature protein part. Thus the signal peptides never relates to the activity.

The DNA having a nucleotide sequence shown in SEQ ID NO. 3 or 7 may be prepared according to the following methods, that is:
(i) by isolating mRNA from a cell which products the polypeptide of the present invention (e.g., human pro-B cell line),
(ii) by preparing a first strand (single strand cDNA) from mRNA thus obtained, followed by preparing a second strand (double strand cDNA) (synthesis of cDNA),
(iii) by inserting cDNA thus obtained into a proper phage vector,
(iv) by transfecting recombinant phage into host cells (construction of cDNA library),
(v) by screening with plaque hybridization a cDNA library using mouse SDF-1 cDNA as a probe,
(vi) by preparing phage DNA containing positive clone obtained, followed by subcloning cDNA which is cut out into a plasmid vector and followed by preparing the restriction enzyme map.
(vii) by determining nucleotide sequence each fragment cut with restriction enzyme, followed by assembling the sequence of full length.

Explained in detail, step (i) may be carried out in accordance with the method of Okayama, H et al (described in method in Enzymology, vol. 154, p3, 1987) after a human pro-B cell line is stimulated by a proper stimulant (e.g. IL-1 etc.) or without stimulation.

Examples of the cells which secrete the polypeptides of the present invention is preferably human pro-B cell line FLEB14. This human cell line FLEB14 may be supplied by 1st lecture, medicinal chemistry, school of medicine, Kyoto University.

Step (ii), (iii) and (iv) are a series of steps for preparing a cDNA library, and may be carried out in accordance with the method of Glubler & Hoffman (Gene, vol. 25, pp. 263, 1983) with a slight modification.

As examples of the vector used in the step (iii), many plasmid vectors (e.g. pB322, pBluescript etc.), phage vectors (e.g. λgt10, λDASH II etc.) are known, a phage vector λgt10 (43.3 kbp, Stratagene) is preferable.
e).

The host cell used in step (iv) is preferably E. coli NM514 (Stratagene).

The step (v) and (vi) may be carried out in accordance with the method described in Molecular Cloning (written by Sam Brook, Fritsh, E. F. and Maniatis, T., published by Cold Spring Harbor Laboratory Press in 1989.).

The sequencing in the step (vii) may be carried out in accordance with the method of Maxam-Gilbert or the dideoxy termination method.

It is necessary to confirm that cDNA obtained covers complete or almost complete length of intact mRNA. These confirmation may be carried out by Northern analysis using the cDNA as a probe (see Molecular Cloning).

If the size of mRNA obtained from the hybridized band and size of the cDNA are almost same, it will be thought that the cDNA is almost full length.

Once the nucleotide sequences shown in SEQ ID NOs. 2, 3, 6, 7 are determined, DNA of the present invention may be obtained by chemical synthesis, by PCR method or by hybridization making use of a fragment of DNA of the present invention, as a probe. Furthermore, DNA of the present invention may be obtained in a desired amount by transforming with a vector DNA inserted a DNA of the present invention into a proper host, followed by culturing the transformant.

The polypeptides of the present invention (shown in SEQ ID NO. 1 or 5) may be prepared by:
(1) isolating and purifying from an organism or a cultured cell,
(2) chemically synthesizing, or
(3) using a skill of biotechnology,
preferably, by the method described in (3).

Examples of expression system when preparing a polypeptide by using a skill of biotechnology, is, for example, the expression system of bacteria, yeast, insect cell and mammalian cell.

For example, the expression in E. coli may be carried out by adding the initiation codon (ATG) to 5' end of a DNA encoding the mature protein, connecting the DNA thus obtained to the downstream of a proper promoter (e.g., trp promoter, lac promoter, IPL promoter, T7 promoter etc.), and then inserting it into a vector (e.g., pBR322, pUC18, pUC19 etc.) which functions in an E. coli strain to prepare an expression vector.

When a signal peptide of bacteria (e.g., signal peptide of pel B) is utilized, the desired polypeptide may be also produced in periplasm. Furthermore, a fusion protein with other polypeptide may be also produced easily.

Furthermore, the expression in a mammalian cell may be carried out, for example, by inserting the DNA shown in SEQ ID NO. 3 or 6 into the downstream of a proper promoter (e.g., SV40 promoter, LTR promoter, metallothionein promoter etc.) in a proper vector (e.g., retrovirus vector, papilloma virus vector, vaccinia virus vector, SV40 vector, etc.) to obtain an expression vector, and transfecting a proper mammalian cell (e.g., monkey COS-7 cell, Chinese hamster CHO cell, mouse L cell etc.) with the expression vector thus obtained, and then culturing the transformant in a proper medium to get a desired polypeptide in the culture medium. The polypeptide thus obtained may be isolated and purified by conventional biochemical methods.

### Effects of the Invention

The polypeptides of the present invention is produced and secreted in pro-B cells, so it may be used for diseases relating to undergrown or abnormal proliferation of hematopoietic cells, neuronal enhancement or depression, immunological enhancement and depression, for example, inflammatory diseases (rheumatoid arthritis, ulcerative colitis etc.), hematopoietic stemcytopenia after bone marrow transplantation, leukocytopenia, thrombocytopenia, B lymphopenia and T lymphopenia after chemotherapy, anemia, infectious diseases, cancer, leukocytosis, AIDS, neurodegenerative diseases (Alzheimer, multiple sclerosis etc.), prevention or treatment of neuronal injury, prevention or treatment of disorder of bone metabolism (osteoporosis etc.) or tissue repair.

In above activities, it was confirmed that the mouse SDF-1α stimulated the proliferation of mouse myeloid progenitor cell line DA1G in the laboratory test. It was suggested that the human SDF-1α also have the same activity.

Further, polyclonal or monoclonal antibodies against the polypeptide of the present invention can be used in the determination of the amount of the said polypeptide in organism, and thereby, may be utilized for the purpose of investigating the relationship between the said polypeptide and diseases, or for the purpose of diagnosing diseases, and the like. Polyclonal and monoclonal antibody thereof may be prepared by conventional methods by using the said polypeptide or the fragment thereof as an antigen.

The DNA of the present invention may be utilized as an important and essential template in preparing the polypeptide of the present invention which is expected to possess various use or for diagnosis of and in the treatment of gene diseases (the treatment of gene defect disease and the treatment by inhibiting expression of the polypeptide by antisense DNA (RNA), and the like). Further, genomic DNA may be isolated by using the DNA of the present invention as a probe. Similarly, it is possible to isolate genes having high homology to the DNA of the present invention in human or those of other species.

### Application for Pharmaceuticals

The polypeptides of the present invention is produced and secreted in pro-B cells, so it may be used for diseases relating to undergrown or abnormal proliferation of hematopoietic cells, neuronal enhancement or depression, immunological enhancement and depression, for example, inflammatory diseases (rheumatoid arthritis, ulcerative colitis etc.), hematopoietic stemcytopenia after bone marrow transplantation, leukocytopenia, thrombocytopenia, B lymphopenia and T lymphopenia after chemotherapy, anemia, infectious diseases, cancer, leukocytosis, AIDS, neurodegenerative diseases (Alzheimer, multiple sclerosis etc.), prevention or treatment of neuronal injury, prevention or treatment of disorder of bone metabolism (osteoporosis etc.) or tissue repair.

The polypeptides of the present invention may be normally administered systemically or partially, usually by oral or parenteral administration, preferably orally, intravenously or intraventricularly.

The doses to be administered are determined depending upon age, body weight, symptom, the desired therapeutic effect, the route of administration, and the duration of the treatment etc. In the human adult, the doses per person per dose are generally between 100 µg and 100 mg, by oral administration, up to several times per day, and between 10 µg and 100 mg, by parenteral administration up to several times per day.

As mentioned above, the doses to be used depend upon various conditions. Therefore, there are cases in which doses lower than or greater than the ranges specified above may be used.

Administration of the compounds of the present invention, may be as solid compositions, liquid compositions or other compositions for oral administration, as injections, liniments or suppositories etc. for parenteral administration.

Solid compositions for oral administration include compressed tablets, pills, capsules, dispersible powders, granules. Capsules include soft capsules and hard capsules.

In such compositions, one or more of the active compound(s) is or are admixed with at least one inert diluent (such as lactose, mannitol, glucose, hydroxypropyl cellulose, microcrystalline cellulose, starch, polyvinylpyrrolidone, magnesium metasilicate aluminate, etc.). The compositions may also comprise, as is normal practice, additional substances other than inert diluents: e.g. lubricating agents (such as magnesium stearate etc.), disintegrating agents (such as cellulose calcium glycolate, etc.), stabilizing agents (such as human serum albumin, lactose etc.), and assisting agents for dissolving (such as arginine, asparaginic acid etc.).

The tablets or pills may, if desired, be coated with a film of gastric or enteric material (such as sugar, gelatin, hydroxypropyl cellulose or hydroxypropylmethyl cellulose phthalate, etc.), or be coated with more than two films. And further, coating may include containment within capsules of absorbable materials such as gelatin.

Other compositions for oral administration included spray compositions which may be prepared by known methods and which comprise one or more of the active compound(s). Spray compositions may comprise additional substances other than inert diluents: e.g. stabilizing agents (sodium sulfite etc.), isotonic buffer (sodium chloride, sodium citrate, citric acid, etc.). For preparation of such spray compositions, for example, the method described in the United States Patent No. 2,868,691 or 3,095,355 (herein incorporated in their entireties by reference) may be used.

Injections may comprise additional other than inert diluents: e.g. preserving agents, wetting agents, emulsifying agents, dispersing agents, stabilizing agent (such as human serum albumin, lactose, etc.), and assisting agents such as assisting agents for dissolving (arginine, asparaginic acid, etc.).

They may be sterilized for example, by filtration through a bacteria-retaining filter, by incorporation of sterilizing agents in the compositions or by irradiation. They may also be manufactured in the form of sterile solid compositions, for example, by freeze-drying, and which can be dissolved in sterile water or some other sterile diluents for injection immediately before used.

Other compositions for parenteral administration include liquids for external use, and endermic liniments (ointment, etc.), suppositories for rectal administration and pessaries which comprise one or more of the active compound(s) and may be prepared by known methods.

### Examples

The following examples are illustrated, but not limit, the present invention.

### Example 1: Northern analysis of human cell line FLEB14

Human pro-B cell line FLEB14 cells (See Katamine, S., et al. Nature, 309, 369 (1984)) were homogenated. The homogenate was incubated with oligo-dT cellulose. Poly(A) RNA was eluted after washing out (Vennstorm, B. et al Cell,28, 135(1982)). 1 µg of poly(A) RNA was electrophoresed in a 1.0% agarose gel and then blotted to a nitrocellulose membrane.

The membrane was hybridized with the 32P-labeled mouse SDF-1 (described as seq. ID No. 3 in Japanese Patent Application No.5-22098; the sequence is shown in seq. ID No. 9.; the factor is now called SDF-1α, as another SDF-1 was found from mouse.) cDNA with 50% formamide, 5 X SSC, 0.1% SDC, 0.1%SDS, 5 X Denhaldt's, 0.1 mg/ml Salmon sperm DNA at 39 °C and washed with 0.3M NaCl, 30mM Na citrate, 0.1% SDS at 50 °C and then autoradiogramed. 3.5 kb and 1.9 kb mRNA were hybridized.

### Example 2 : preparation of cDNA from mRNA of human pro-B cell line

A cDNA library was constructed from human pro-B cell line FLEB14 cells by conventional method (See Molecular Cloning ; Sambrook, J., Fritsh, E. F., & Maniatis, T, Cold Spring Harbor Laboratory Press (1989)). cDNA was synthesized using Time Saver cDNA synthesis kit (Pharmacia).

First strand was synthesized from FLEB14 poly(A)-RNA (5 µg) using a reverse transcriptase and an oligo-dT primer. And using a DNA polymerase I, double strand cDNA was synthesized.

cDNA was ligated an EcoRI-NotI adapter: and then phosphorylated, cDNA which are larger than 800 bp were recovered from a 0.8 % agarose gel with a glass powder (Geneclean II DNA purification kit, available from Bio101).

### Example 3 : Preparation of cDNA library and cross hybridization

cDNA obtained was ligated to a λgt10 phase vector (available from Stratagene) which have EcoRl arm treated with phosphatase.

In vitro packaging was followed by the protocol of in vitro packaging kit LAMDA INN (available from Nihon gene). The recombinant phages were transfected to host E. Coli NM514 (available from Stratagene). A cDNA library containing 1 x 10 6 plaques was obtained. 1 x 10 6 λgt10 phage plaques of the cDNA library were transfected to nitrocellulose membranes. The membranes were hybridized with the 32P-labeled mouse SDF-1α cDNA (shown in seq. ID no.9, the same cDNA used in Example 1) in 50% formamide, 5 X SSD, 0.1 % SDS, 5 X Denhaldt's 0.1 mg/ml Salmon sperm DNA, at 39°C and washed in 0.3 M NaCl, 30 mM Na citrate, 0.1 % SDS at 50 °C and autoradiogramed. 40 positive clones were obtained.

### Example 4 : Isolation of positive clones

Phage DNA were prepared from 9 positive clones by the conventional method (See Cell Technology Experimental Protocol, pp. 8, published by Shuujun-sha). Phage DNA was digested at Not I. The length of insert cDNA were measured by agarose gel electrophoresis. The length of 8 clones were 1.9kb, and the length of one clone was 3.5kb. It was thought that these two types clones are almost full length of human SDF-1α and SDF-β cDNA from the result of Northern analysis.

cDNA from one clone was picked up from 8 clones of 1.9 kb length, and one clone of 3.5 kb length were digested at Not I were subjected to agarose electrophoresis, and the fragments were cut out and were subcloning at Not I site of plasmid pBluescript.

### Example 5 : Preparation of restriction enzyme map and sequencing

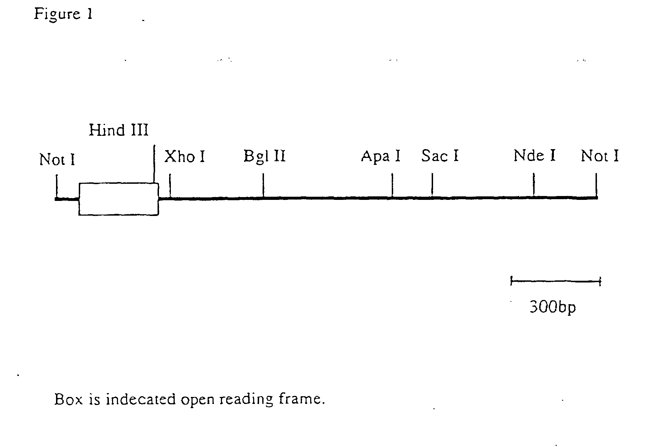

A restriction enzyme map of human SDF-1 (1.9kb) was prepared (shown in Fig. 1). Each fragments of restriction enzyme were cDNA subcloned into pBluescript, followed by determined about 300 bp nucleotide sequences of both ends of each inserts. Assembling these sequences, nucleotide sequences of full length were determined (shown in Seq. ID. No. 3).

An open reading frame and an amino acid sequence were determined from the nucleotide sequence of full length cDNA, with the results shown in Seq. ID No. 1. In comparison with known signal peptides, 30 - 40 amino acids of N-termini obtained was compared with known signal peptide, a signal peptide of the polypeptides of the present invention was presumed and obtained sequence shown in seq. ID No. 4 (See Von Heuane, G. Nucleic Acids Res. 14 ,4683 (1986)).

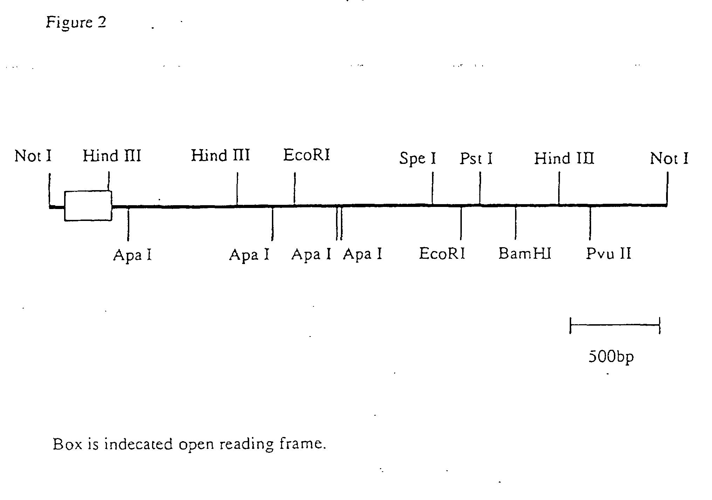

By the same procedure as described above, a restriction enzyme map (shown in Figure 2), full length nucleotide sequences (shown in seq. ID No. 7), an open reading frame (shown in seq. ID No. 6), an amino acid sequence (shown in seq. ID No. 5) and a sequence shown with signal peptide (shown in seq. ID No. 8) of 3.5 kb clone were obtained.

Deduced amino acid sequences of 3.5 kb clone and 1.9 kb clone were very similar each other, so 1.9 kb clone was named SDF-1α and 3.5 kb clone was named SDF-1β.

Nucleotide sequences were determined by cycle sequence method using fluorescence determinator (supplied by Applied Biosystem Inc.). Reading of nucleotide sequences were carried out by DNA sequencer (Model 373, supplied by Applied Biosystem Inc.).

Nucleotide sequences and deduced amino acid sequences of SDF-1α and 1β were homology searched by a computer in data base (GENBANK and EMBL for DNA, NBRF and SWISSPROT for amino acid sequence). it was confirmed that cDNAs of the present invention are encoding novel peptides.

### Example 6 : Construction of plasmid vector for using the preparation of expression vector

As an expression vector, pUC-SRαML-1 (This vector is disclosed itself and preparation thereof in European Patent publication No. 559428) derivative was used. This derivative was constructed to insert two kinds of fragments as shown below: between Pstl and Sacl and between Spel and Kpnl site in the multi-cloning site, respectively.

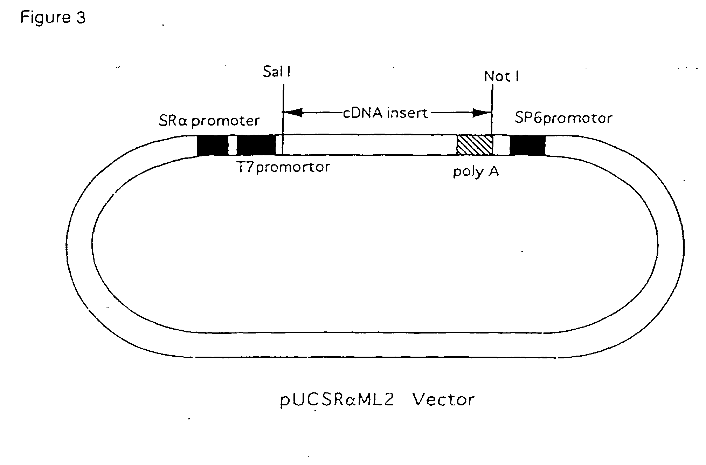

The pUC-SRαML1 vector was digested with Pstl and Sacl and the resulting digest was subjected to agarose gel electrophoresis to prepare and recover an about 4.1 kbp fragment and thereafter removing the 5'-end phosphoric acid group by BAP (bacterial alkaline phosphatase) treatment. The phosphorylated DNA fragment T7 was ligated with the thus prepared about 4.1 kbp fragment from pUC-SRαML1 to make them into a circular form. The resulting vector was, moreover, digested with Spel and Kpnl and the resulting digest was subjected to agarose gel electrophoresis to prepare and recover an about 4.1 kbp fragment and thereafter removing the 5'-end phosphoric acid group by BAP (bacterial alkaline phosphatase) treatment. The phosphorylated DNA fragment SP6 was ligated with the thus prepared about 4.1 kbp fragment to make them into a circular form. The plasmid vector constructed in this manner was named pUC-SRαML2 (See Figure 3).

### Example 7 : Construction of expression vector

Regarding hSDF-1α, primer X, Y and YH were synthesized. Sequences of primer X, Y and YH are as follows:

The hSDF-1α plasmid was subjected to PCR using the thus synthesized oligonucleotides X and Y as primers. The thus obtained PCR fragment contains a sequence placed 5'-adjacent to the initiation codon, that is corresponding to Kozac sequence which is known among skilled in the art, and cDNA which encodes a protein molecule consisting of the hSDF-1α protein. The PCR fragment was digested with Sall and SpeI and the resulting digest was separated and purified and then inserted into the Sall -SpeI site of the pUC-SRαML2 prepared in example 6 to obtain an expression vector pUC-SRαML2 - hSDF-1αA.

Moreover, the hSDF-1α plasmid was subjected to PCR using the synthesized oligonucleotides X and YH as primers. The thus obtained PCR fragment contains a sequence placed 5'-adjacent to the initiation codon, that is corresponding to Kozac sequence which is known among skilled in the art, and cDNA which encodes a protein molecule consisting of the hSDF-1α protein and six additional histidine (His) residues attached to its C-terminal end. The PCR fragment was digested with SalI and spei and the resulting digest was separated and purified and then inserted into the Sall - SpeI site of the pUC-SRαML2 prepared in example 6 to obtain an expression vector pUC-SRαML2 - hSDF-1αB.

As for hSDF-1β, primer Z and ZH were synthesized. Sequences of primer Z and ZH are as follows:

The hSDF-1β plasmid was subjected to PCR using the thus synthesized oligonucleotides X and Z as primers. The thus obtained PCR fragment contains a sequence placed 5'-adjacent to the initiation codon, that is corresponding to Kozac sequence which is known among skilled in the art, and cDNA which encodes a protein molecule consisting of the hSDF-1β protein. The PCR fragment was digested with Sall and SpeI and the resulting digest was separated and purified and then inserted into the SalI - SpeIsite of the pUC-SRαML2 prepared in example 6 to obtain an expression vector pUC-SRαML2 - hSDF-1βA.

Moreover, the hSDF-1β plasmid was subjected to PCR using the synthesized oligonucleotides X and ZH as primers. The thus obtained PCR fragment contains a sequence placed 5'-adjacent to the initiation codon, that is corresponding to Kozac sequence which is known among skilled in the art, and cDNA which encodes a protein molecule consisting of the hSDF-1β protein and six additional histidine (His) residues attached to its C-terminal end. The PCR fragment was digested with Sall and SpeI and the resulting digest was separated and purified and then inserted into the Sall - SpeI site of the pUC-SRαML2 prepared in example 6 to obtain an expression vector pUC-SRαML2 - hSDF-1βB.

Each of the thus constructed pUC-SRαML2-hSDF-1αA, pUC-SRαML2-hSDF-1αB, pUC-SRαML2-hSDF-1βA and pUC-SRαML2-hSDF-1β plasmids were transfected into an E. coli strain DH5, recovered from a 100 ml culture of the resulting transformant and then purified by CsCl density gradient centrifugation twice.

### Example 8: Expression in COS cells

Each of the plasmid DNA preparations pUC-SRαML2, pUC-SRαML2-hSDF-1αA, pUC-SRαML2-hSDF-1αB, pUG-SRαML2-hSDF-1βA and pUC-SRaML2-hSDF-1 1βB were introduced into COS-7 cells (Cell, vol. 23, p. 175, 1981) by means of the diethylaminoethyl (DEAE) dextran method (J. Immunology, vol. 136, p. 4291, 1986).

That is, about 1.8 x 10⁶ COS-7 cells were inoculated into a 225 cm² capacity flask (manufactured by Corning) together with 50 ml of a liquid culture medium (Dulbecco's modified MEM medium supplemented with 10% decomplemented fetal bovine serum). After overnight incubation in a carbon dioxide incubator (37°C, 5% CO2) and subsequent removal of the culture supernatant, 12 ml of a DNA cocktail (Dulbecco's modified MEM medium supplemented with 15 µg of each plasmid DNA, 50 mM Tris-HCl buffer (pH 7.4) and 400 µg/ml of DEAE-dextran) was added to each flask and culture was carried out for 3 hours at 37 °C in an atmosphere of 5% CO2. Thereafter, the DNA cocktail was replaced by 15 ml of a chloroquine solution (Dulbecco's modified MEM medium supplemented with 150 µM chloroquine and 7% decomplemented fetal bovine serum), followed by additional 3 hours of culture.

After removing the chloroquine solution, the aforementioned liquid culture medium (50 ml) was added to each of the resulting flasks which were then incubated at 37 °C in an atmosphere of 5% CO2 for 72 hours to find growth of the cells in each flask into almost monolayer form. After removing the culture supernatant, the cells in each flask were washed with a serum-free liquid culture medium (trade name, SFM-101; available from Nissui Pharmaceutical Co., Ltd.) and then supplied with 75 ml of the same serum-free liquid culture medium, and the culturing was continued for another 72 hours. Thereafter, the resulting culture supernatant was recovered and filtered through a membrane filter (trade name, STERIVEX-GS; available from Millipore Corp.) to remove the cells and cell debris. The thus obtained culture supernatant samples were stored at 4 °C for future use. A culture supernatant of COS cells which have been transformed with plasmid containing the hSDF-1α and β cDNA inserts are expected to contain expressed and secreted mature protein moieties of polypeptides which correspond to hSDF-1α and β.

### Example 9 : Confirmation of expression

A 2 ml portion of each of the culture supernatants of transformed COS cells obtained in Example 8 was concentrated to a volume of 100 ml using a centrifugal concentration filter (trade name, Centricon-10; available from Millipore Corp.). A 1 µl portion of each of the thus concentrated samples was mixed with the same volume of a loading buffer (0.125 M Tris-HCl buffer (pH 6.8), 4% sodium dodecyl sulfate and 30% glycerol) for SDS-PAGE (sodium dodecyl sulfate polyacrylamide gel electrophoresis) use, and the mixture was treated at 90 °C for 3 minutes and then subjected to SDS-PAGE.

In the case of the hSDF-1αB and βB proteins having His hexamer introduced to the C-terminus of the proteins, not only their corresponding COS cell culture supernatant but also their purified products were subjected to the SDS-PAGE analysis.

Purification of the protein was carried out by means of a metal chelate affinity chromatography (Biotechnology, vol. 9, p. 273, 1991), making use of the function of His to form complex compounds with various transition metal ions. That is, a culture supernatant (350 ml) obtained from COS cells was mixed with a sodium chloride aqueous solution in such an amount that the final concentration of the salt became 1 M, and the resulting mixture was applied to a column packed with 4 ml of a zinc-linked chelating Sepharose (trade name, Chelating Sepharose Fast-Flow; available from Pharmacia) to adsorb the protein to the resin. The column was washed with 50 mM phosphate buffer (pH 7.0) containing 1 M sodium chloride aqueous solution (40 ml), and the protein retained in the column was eluted with 50 mM phosphate buffer (pH 7.0) containing 1 M sodium chloride aqueous solution and 0.4 M imidazole. Thereafter, the resulting elute was concentrated to a volume of 100 µl, and a portion of the concentrated sample was subjected to SDS-PAGE analysis. The SDS-PAGE analysis was carried out using a SDS 10/20 gradient gel and a product which corresponds to a molecular weight of hSDF-1α and SDF-1β was detected respectively.

## Claims

1. A polypeptide having the amino acid sequence shown in SEQ ID No. 1.

2. DNA having the nucleotide sequence shown in SEQ ID No. 2.

3. DNA having the nucleotide sequence shown in SEQ ID No. 3.

4. A replication and expression vector comprising DNA according to any one of claims 2 or 3 .

5. Host cells transformed or transfected with a replication and expression vector according to claim 4.

6. A method of producing a polypeptide which comprises culturing host cells according to claim 5 under conditions effective to express a polypeptide according to claim 1.

7. A pharmaceutical composition containing a polypeptide according to claim 1 in association with a pharmaceutically acceptable diluent and/or carrier.

8. A polypeptide having the amino acid sequence shown in SEQ ID No. 5.

9. DNA having the nucleotide sequence shown in SEQ ID No. 6.

10. DNA having the nucleotide sequence shown in SEQ ID No. 7.

11. A replication and expression vector comprising DNA according to any one of claims 9 or 10.

12. Host cells transformed or transfected with a replication and expression vector according to claim 11.

13. A method of producing a polypeptide which comprises culturing host cells according to claim 12 under conditions effective to express a polypeptide according to claim 8.

14. A pharmaceutical composition containing a polypeptide according to claim 8 in association with a pharmaceutically acceptable diluent and/or carrier.

15. A polypeptide having a region ranging from 22-position to 89-position in the amino acid sequence shown in SEQ ID No. 1.

16. A polypeptide having a region ranging from 22-position to 93-position in the amino acid sequence shown in SEQ ID No. 5.

## Patentansprüche

1. Polypeptid mit der Aminosequenz, die in SEQ ID No. 1 angegeben ist.

2. DNA mit der Nucleotidsequenz, die in SEQ ID No. 2 angegeben ist.

3. DNA mit der Nucleotidsequenz, die in SEQ ID No. 3 angegeben ist.

4. Replikations- und Expressionsvektor, der DNA gemäß einem der Ansprüche 2 oder 3 umfasst.

5. Wirtszellen, die mit einem Replikations- und Expressionsvektor gemäß Anspruch 4 transformiert oder transfiziert sind.

6. Verfahren zur Herstellung eines Polypeptids, das das Kultivieren von Wirtszellen gemäß Anspruch 5 unter zur Expression eines Polypeptids gemäß Anspruch 1 wirksamen Bedingungen umfasst.

7. Pharmazeutische Zusammensetzung, die ein Polypeptid gemäß Anspruch 1 in Verbindung mit einem pharmazeutisch akzeptablen Verdünnungsmittel und/oder Träger enthält.

8. Polypeptid mit der Aminosequenz, die in SEQ ID No. 5 angegeben ist.

9. DNA mit der Nucleotidsequenz, die in SEQ ID No. 6 angegeben ist.

10. DNA mit der Nucleotidsequenz, die in SEQ ID No. 7 angegeben ist.

11. Replikations- und Expressionsvektor, der DNA gemäß einem der Ansprüche 9 oder 10 umfasst.

12. Wirtszellen, die mit einem Replikations- und Expressionsvektor gemäß Anspruch 11 transformiert oder transfiziert sind.

13. Verfahren zur Herstellung eines Polypeptids, das das Kultivieren von Wirtszellen gemäß Anspruch 12 unter zur Expression eines Polypeptids gemäß Anspruch 8 wirksamen Bedingungen umfasst.

14. Pharmazeutische Zusammensetzung, die ein Polypeptid gemäß Anspruch 8 in Verbindung mit einem pharmazeutisch akzeptablen Verdünnungsmittel und/oder Träger enthält.

15. Polypeptid mit einer Region, die von Position 22 bis Position 89 in der in SEQ ID No. 1 angegebenen Aminosäuresequenz reicht.

16. Polypeptid mit einer Region, die von Position 22 bis Position 93 in der in SEQ ID No. 5 angegebenen Aminosäuresequenz reicht.

## Revendications

1. Polypeptide ayant la séquence d'aminoacides présentée dans SEQ ID n°: 1.

2. ADN ayant la séquence nucléotidique présentée dans SEQ ID n°: 2.

3. ADN ayant la séquence nucléotidique présentée dans SEQ ID n°: 3.

4. Vecteur de réplication et d'expression comprenant un ADN selon l'une quelconque des revendications 2 ou 3.

5. Cellules hôtes transformées ou transfectées avec un vecteur de réplication et d'expression selon la revendication 4.

6. Méthode de production d'un polypeptide qui comprend la culture de cellules hôtes selon la revendication 5 dans des conditions efficaces pour exprimer un polypeptide selon la revendication 1.

7. Composition pharmaceutique contenant un polypeptide selon la revendication 1 en association avec un diluant et/ou un support pharmaceutiquement acceptables.

8. Polypeptide ayant la séquence d'aminoacides présentée dans SEQ ID n°: 5.

9. ADN ayant la séquence nucléotidique présentée dans SEQ ID n°: 6.

10. ADN ayant la séquence nucléotidique présentée dans SEQ ID n°: 7.

11. Vecteur de réplication et d'expression comprenant un ADN selon l'une quelconque des revendications 9 ou 10.

12. Cellules hôtes transformées ou transfectées avec un vecteur de réplication et d'expression selon la revendication 11.

13. Méthode de production d'un polypeptide qui comprend la culture de cellules hôtes selon la revendication 12 dans des conditions efficaces pour exprimer un polypeptide selon la revendication 8.

14. Composition pharmaceutique contenant un polypeptide selon la revendication 8 en association avec un diluant et/ou un support pharmaceutiquement acceptables.

15. Polypeptide ayant une région allant de la position 22 à la position 89 de la séquence des aminoacides présentée dans SEQ ID n°: 1.

16. Polypeptide ayant une région allant de la position 22 à la position 93 de la séquence des aminoacides présentée dans SEQ ID n°: 5.
